# EUROPÄISCHE PATENTSCHRIFT

(11) **EP 1 567 216 B1**
(45) Veröffentlichungstag und Bekanntmachung des Hinweises auf die Patenterteilung: **09.05.2007**
(21) Anmeldenummer: 03811709.9
(22) Anmeldetag: 17.11.2003
(51) Int. Cl.: A61M 5/32

(54) **ABZIEVORRICHTUNG FÜR INJEKTIONSNADELN**
REMOVING DEVICE FOR INJECTION NEEDLES
DISPOSITIF DE RETRAIT D'AIGUILLE D'INJECTION

(30) Priorität: 26.11.2002 DE 10255134
(43) Veröffentlichungstag der Anmeldung: 31.08.2005
(73) Patentinhaber: TecPharma Licensing AG, 3401 Burgdorf (CH)
(72) Erfinder: LEHMANN, Hans-Ulrich, CH-2564 BELLMUND (CH)
(86) Internationale Anmeldenummer: PCT/CH2003/000760
(87) Internationale Veröffentlichungsnummer: WO 2004/047894

(56) Entgegenhaltungen:
- EP-A- 0 787 501
- WO-A-01/87387
- WO-A-97/40869
- DE-A- 2 740 335
- FR-A- 2 760 647
- US-A- 6 036 671
- US-A- 6 062 001

## Beschreibung

Die Erfindung betrifft eine Abziehvorrichtung für Injektionsnadeln von einer Injektions- oder Infusionsvorrichtung, insbesondere einem Injektionspen, und einen Behälter zur Aufbewahrung gebrauchter Nadeleinheiten z. B. eines Injektionspens.

Injektionspens werden zur subkutanen Verabreichung eines Wirkstoffes verwendet. Dabei sollte die Anwendung des Pens und die Dosierung des Wirkstoffs derart einfach und sicher ausgebildet sein, dass die Verabreichung nicht nur von medizinisch geschultem Personen, sondern auch von Laien wie z. B. dem Patienten selbst durchgeführt werden kann. Die Einstellung der erforderlichen Dosismenge, das Einstechen einer Injektionsnadel in den ausgewählten Injektionsbereich und die Abgabe des Wirkstoffs kann mit einem bekannten Pen durch einfache Handbewegungen erfolgen.

Jede Injektionsnadel oder Kanüle sollte im Allgemeinen jedoch nur einmal für eine Injektion verwendet werden, während der Injektionspen wiederholt eingesetzt und auch nachgeladen werden kann. Eine Injektionsnadel ist deshalb meist in einer Nadeleinheit angeordnet, welche nach dem Gebrauch der Nadel von dem Injektionspen abgenommen werden kann, so dass eine neue Nadeleinheit für die nächste Verabreichung in den Pen eingesetzt werden kann.

Das Abziehen der Nadeleinheit birgt jedoch eine gewisse Verletzungsgefahr. Die Injektionsnadel liegt nach der Verabreichung des Wirkstoffes frei und eine möglicherweise vorgesehene Schutzhülle muss erst aufgebracht werden.

Aus der US 5,873,462 und der US 5,829,589 ist jeweils ein Injektionsnadelbehälter zur Aufbewahrung von Nadeleinheiten bekannt, der für jede Nadeleinheit einen eigenen zu einer Seite offenen Hohlraum aufweist, aus dem eine Nadeleinheit entnommen und in den sie wieder abgegeben werden kann. Für das Rückführen der Nadeleinheit nach ihrem Gebrauch in den Hohlraum des Behälters ist nach der US 5,873,462 eine Sperreinrichtung vorgesehen, die an der Öffnung des Hohlraums einen Verschlussflansch und an der Nadeleinheit einen Verschlussring aufweist. Beim Einsetzen der Nadeleinheit mit Hilfe des Injektionspens in den Hohlraum gehen der Flansch und der Ring eine feste Verbindung ein, so dass die Nadeleinheit von dem Pen abgezogen werden kann. In dem Hohlraum ist auf geeigneter Höhe eine nach innen verlaufende Schulter ausgebildet, auf der die Nadeleinheit beim Einsetzen aufsitzt. Die Nadeleinheit wird durch die Sperreinrichtung und die Schulter fest in ihrer Position in dem Hohlraum gehalten. In der US 5,829,589 ist die Sperreinrichtung für die Nadeleinheit in dem Hohlraum als Schraubverbindung ausgebildet.

Die Erfindung hat es sich zur Aufgabe gemacht, eine Abziehvorrichtung für Injektionsnadeln zu schaffen, die das Abziehen einer Nadeleinheit von einem Injektionspen erleichtert, die dabei entstehende Verletzungs- und Infektionsgefahr verringert und einen Kontakt mit der gebrauchten Nadeleinheit verhindert.

Ferner ist es die Aufgabe der Erfindung einen Behälter zur Aufbewahrung gebrauchter Nadeleinheiten bereitzustellen, in den eine Nadeleinheit auf sichere und einfache Weise eingebracht werden kann, ohne das Risiko der Verletzung oder Infizierung beim Entfernen der gebrauchten Nadeleinheit von dem Injektionspen und beim Einfüllen in den Behälter.

Die Aufgabe der Erfindung wird durch eine Vorrichtung zum Abziehen einer Nadeleinheit und einen Behälter mit einer solchen Vorrichtung nach dem unabhängigen Anspruch gelöst. Vorteilhafte Ausgestaltungen gehen aus den Unteransprüchen hervor.

Nach der vorliegenden Erfindung umfasst eine Vorrichtung zum Abziehen einer Nadeleinheit von einer Injektionsvorrichtung wie einem Injektionspen eine Öffnung zum Aufnehmen der Nadeleinheit und ein Sperrmittel. Das Sperrmittel ist in wenigstens einem Randbereich entlang des Umfangs der Öffnung derart ausgebildet, dass die Nadeleinheit nach dem Einführen in die Öffnung in Einführrichtung beweglich ist. Bei einer Bewegung der Nadeleinheit entgegen der Einführrichtung geht die Nadeleinheit jedoch mit dem Sperrmittel in der Öffnung eine feste Sperrverbindung ein, so dass sie von dieser gesperrt wird.

Die erfindungsgemäße Abziehvorrichtung stellt ein einfaches und schnelles Entfernen einer Nadeleinheit von einem Injektionspen sicher, indem der Injektionspen durch einen simplen Handgriff von dem Injektionspen abgezogen werden kann. Hierfür ist es lediglich erforderlich das Ende des Injektionspens mit der Nadeleinheit in die Öffnung der Abziehvorrichtung einzuführen. Durch leichtes manuelles Eindrücken wird die Sperrverbindung zwischen der Nadeleinheit und der Sperreinrichtung im Rand der Öffnung hergestellt. Das Herausziehen des Injektionspens erfolgt gegen einen nur geringen Widerstand, der durch das Ablösen der Nadeleinheit von dem Pen entsteht. Diese Widerstandskraft ist geringer als die Kraft mit der die Nadeleinheit in der Sperreinrichtung gehalten wird, so dass sich beim Herausziehen des Injektionspens die Nadeleinheit vom Pen lösen kann. Auf diese Weise kann die Nadeleinheit von dem Injektionspen abgezogen werden, ohne dass ein Anwender dabei mit der Nadeleinheit in Kontakt kommt, da sie während des gesamten Abziehvorgangs innerhalb der Öffnung liegt.

In einem bevorzugten Ausführungsbeispiel erstreckt sich die Sperreinrichtung über den gesamten Umfang des Randes der Öffnung, der vorteilhafterweise auf den Außenumfang einer Nadeleinheit abgestimmt ist. Die Sperreinrichtung kann daher an dem gesamten äußeren Umfang der Nadeleinheit angreifen. Vorzugsweise weist zur Ausbildung der Sperreinrichtung eine in Einführrichtung erste Kante des Randes der Öffnung einen größeren Umfang auf, als eine zweite Kante der Öffnung. Beispielsweise kann die Sperreinrichtung durch einen sich konisch in Einführrichtung verengenden Rand der Öffnung ausgebildet sein. Der Umfang der zweiten Kante der Öffnung ist derart auf den Außenumfang der Nadeleinheit abgestimmt, dass zwischen der Öffnung und der Nadeleinheit eine Klemmverbindung entsteht. Durch den sich konisch verengenden Verlauf des Randes der Öffnung, ist die Nadeleinheit leicht in der Öffnung zu zentrieren und kann gegen einen nur geringen Widerstand in die Sperrverbindung geklemmt werden.

Die zweite Kante wirkt dabei wie eine Art Haltekralle auf die Nadeleinheit. Die Haltekraft der Sperreinrichtung ist dabei gerade so groß dimensioniert, dass der Widerstand gegen das Abziehen der Nadeleinheit aus dem Injektionspen überwunden wird. Im Allgemeinen ist eine Kraft von wenigen Newton ausreichend.

Es ist auch möglich, die Sperreinrichtung durch ein oder mehrere an der Randfläche der Öffnung angeordnete Gummielemente vorzusehen. Beim Eindrücken der Nadeleinheit in die Sperreinrichtung wird der Gummi komprimiert und stellt eine Klemmverbindung mit der Nadeleinheit her.

Alternativ kann die Sperreinrichtung an der Öffnung auch durch ein Rastmittel realisiert sein, das mit einem komplementären Rastmittel an der Nadeleinheit zusammenwirkt. Dadurch können bei einem System aus einer Abziehvorrichtung und einem Injektionspen mit einer Nadeleinheit die Vorteile der Erfindung genutzt werden. Das Rastmittel der Sperreinrichtung kann beispielsweise durch mehrere in Umfangsrichtung an der Randfläche der Öffnung angeordnete Rillen verwirklicht werden. Das komplementäre Rastmittel der Nadeleinheit kann entsprechend durch mehrere in Umfangsrichtung auf der Außenfläche der Nadeleinheit angeordnete Vorsprünge gegeben sein. Dabei ist lediglich darauf zu achten, dass die Sperrverbindung zwischen der Sperreinrichtung und der Nadeleinheit wenigstens durch eine nachfolgend in die Öffnung eingebrachte Nadeleinheit gelöst werden kann und die Haltekraft der Sperrverbindung größer ist als die zum Lösen der Nadeleinheit von dem Injektionspen erforderliche Kraft. Ferner ist es möglich bereits in der Nadeleinheit vorhandene Sichtfenster als Rastmittel zu verwenden und die Rastmittel der Sperreinrichtung, z. B. Noppen in der Umfangsfläche des Randes der Öffnung, auf die Anordnung der Sichtfenster an der Nadeleinheit abzustimmen.

Letztlich kann die Sperreinrichtung durch eine Drehverbindung der Öffnung mit der Nadeleinheit gegeben sein. Vorzugsweise ist hierfür im Rand der Öffnung wenigstens eine, vorzugsweise aber zwei, zumindest teilweise schräg verlaufende Führungsrillen vorgesehen. Entlang der Führungsrille wird ein seitlich an der Nadeleinheit angeordneter Stift durch die Sperreinrichtung geführt, wobei eine Anzahl an Stiften entsprechend der Zahl der Rillen an der Nadeleinheit vorgesehen ist.

Die Führungsrille beginnt an der in Einführrichtung ersten Kante der Randfläche der Öffnung und mündet in eine zweite Kante, so dass für die Stifte der Nadeleinheit eine Eingangs- und ein Ausgangsrillenteil entsteht. Zwischen diesen Rillenteilen ist ein Mittelteil der Rille mit einer Sperrwirkung vorgesehen, der hierfür z. B. gegenüber der Achse des Führungselements eine größere Neigung aufweist als der Eingangs- und Ausgangsteil. Es wäre auch denkbar diesen Rillenteil quer zur Axialrichtung, d. h. in Umfangsrichtung, auszubilden. Der Eingangsteil und der Ausgangsteil der Rille sind daher zu einander versetzt angeordnet und durch den schräg oder quer verlaufenden mittleren Sperrteil der Rille verbunden.

Die Stifte der Nadeleinheit greifen beim Einführen des Injektionspens in die Führungsrillen ein. Durch einen geringen Druck werden die Stifte entlang der Rillen geführt und gegenüber der Eingangsposition verdreht, wobei innerhalb des schrägen Mittelteils der Rille eine Art Rastverbindung entsteht, z. B. durch eine Rückdrehsperre in Form eines Vorsprungs in der Rille. Beim Herausziehen des Injektionspens aus dem Führungselement wird die Nadeleinheit gegen den Widerstand der Raststellung abgezogen und kann entlang dem Ausgangsteil der Rille aus der Sperreinrichtung fallen.

Bei der erfindungsgemäßen Abziehvorrichtung ist ein zylinderförmiges Führungselement mit einer Einführöffnung für den Injektionspen über der Öffnung angeordnet. Das Führungselement weist ein Aufnahmerohr für den Injektionspen auf, das in dem Führungselement verschiebbar angeordnet ist. Das Aufnahmerohr hat ein oberes Ende, das durch die Einführöffnung aus dem Führungselement herausragt und ein unteres Ende, das innerhalb des Führungselementes liegt und bevorzugt nach innen gerichtete Vorsprünge aufweist. An diesen Vorsprüngen sitzt der Injektionspen z. B. mit seinem Gehäuse nach dem Einführen in das Aufnahmerohr derart auf, dass die Nadeleinheit an diesen Vorsprüngen vorbei aus dem Aufnahmerohr ragt und in Richtung auf die Sperreinrichtung in der Öffnung gerichtet wird. Am Außenumfang des Aufnahmerohres können seitliche Vorsprünge angeordnete sein, die an der Innenfläche des Führungselementes anliegen, so dass das Aufnahmerohr von dem Führungselement geführt wird. Dabei wird die axiale Bewegung des Aufnahmerohres entgegen der Einführrichtung durch an der Einführöffnung des Führungselements radial nach innen ragende Kanten begrenzt, an welchen die seitlichen nach außen ragenden Vorsprünge des Aufnahmerohrs anstoßen. In Einführrichtung wird die Bewegung durch das Anstoßen des unteren Endes des Aufnahmerohrs an der Abziehvorrichtung begrenzt, wobei die Nadeleinheit in die Sperreinrichtung in der Öffnung der Abziehvorrichtung eingreift und dann mit dieser eine Sperrverbindung eingeht.

Durch das Führungselement und das Aufnahmerohr kann der Injektionspen genau über und innerhalb der Sperreinrichtung positioniert werden. Das Einführen des Pens in die Sperreinrichtung wird dadurch erleichtert und die Sperrverbindung erfordert durch die exakte Führung weniger Druckkraft.

Es ist vorteilhaft zwischen dem Aufnahmerohr und dem Führungselement ein Vorspannelement, wie z. B. eine Spiralfeder, anzuordnen. Die Spiralfeder kann mit ihrem einen Ende an einem unteren Ende des Führungselements und mit ihrem anderen Ende an den seitlichen Vorsprüngen des Aufnahmerohrs angreifen. Nach dem Einführen des Injektionspens in das Aufnahmerohr und beim Weiterführen des Aufnahmerohrs innerhalb des Führungselements wird die Spiralfeder komprimiert bis die Nadeleinheit in die Sperreinrichtung eingreift. Beim Herausziehen des Injektionspens muss nur die Andrückkraft reduziert werden und das Abziehen der Nadeleinheit von dem Pen erfolgt durch die Federkraft der Spiralfeder, so dass das Entfernen der Nadeleinheit von dem Injektionspen erleichtert wird.

Alternativ zu der Spiralfeder kann hierfür in der Wand des Führungselements eine Hebelanordnung zur Betätigung des Aufnahmerohrs vorgesehen werden. Der Hebel kann vorzugsweise in einem unteren Bereich durch die Wand des Führungselements angeordnet sein. Das innen liegende Ende des Hebels kann beim Eingreifen der Nadeleinheit in die Sperreinrichtung z. B. an einem unteren Bereich des Aufnahmerohrs angreifen. Das außen liegende Ende des Hebels ist dabei derart von außen betätigbar, dass das Aufnahmerohr wieder aus dem Führungselement heraus geschoben werden kann. Durch einen solchen Hebel kann das Abziehen der Nadeleinheit weiter erleichtert werden.

Die Aufgabe der vorliegenden Erfindung wird ferner durch einen Behälter zur Aufbewahrung wenigstens einer gebrauchten Nadeleinheit eines Injektionspens gelöst, wobei der Behälter eine Einfüllöffnung aufweist, die mit einer Vorrichtung zum Abziehen der Nadeleinheit von dem Injektionspen versehen ist, wie sie bisher beschrieben wurde. In einen solchen Behälter kann eine Nadeleinheit eingebracht werden, ohne dass, der Anwender mit der Nadel in Berührung kommt. Die Abgabe einer gebrauchten Nadeleinheit von dem Injektionspen in den Behälter zur weiteren Entsorgung, ist durch einen einfachen Handgriff möglich und kann daher schnell und effizient durchgeführt werden.

Bei einem erfindungsgemäßen Behälter ist es zum einen möglich, dass nach dem Herausziehen des Injektionspens aus der Abziehvorrichtung sich die Sperrverbindung der Nadeleinheit mit der Sperreinrichtung löst und die Nadeleinheit freigegeben wird, so das sie lose in dem Behälter verbleibt. Zum anderen kann die Nadeleinheit derart von dem Injektionspen gelöst werden, dass sie nach dem Herausziehen des Injektionspens aus dem Führungselement mit der Sperreinrichtung in Sperrverbindung verbleibt und erst von einem nachfolgend in den Behälter eingeführten Injektionspen aus der Sperrverbindung gestoßen wird. Natürlich kann ein Behälter auch so dimensioniert sein, dass er nur eine Nadeleinheit aufnehmen kann.

Der Behälter kann besonders vorteilhaft ein System mit einem Container bilden, der den Behälter aufnimmt und einen Depotraum für unbenutzte Nadeleinheiten aufweist. Als Container kann z. B. eine konventionelle Schachtel oder eine Kartonbox verwendet werden. Die Höhe des Containers ist bevorzugt auf die Höhe des Behälters abgestimmt, so dass beim Öffnen des Containers der Behälter leicht zugänglich ist. Wird der Behälter in dem Container untergebracht, verbleibt in dem Container genügend Raum, in dem unbenutzte Nadeleinheiten als Vorrat aufbewahrt werden können. Bevorzugt sind das Fassungsvolumen des Behälters für gebrauchte Nadeleinheiten und das Fassungsvolumen des Depotraums für unbenutzte Nadeleinheiten aufeinander abgestimmt. Das heißt der Depotraum sollte nicht eine größere Anzahl an unbenutzte Nadeleinheiten aufnehmen können als gebrauchte Nadeleinheiten in dem Behälter entsorgt werden können. Dadurch bildet das System aus Behälter und Container eine praktische Versorgungs- und Entsorgungseinrichtung für Nadeleinheiten.

Bevorzugte Ausführungsbeispiele der Erfindung werden nachfolgend anhand der Zeichnung erläutert. In dieser stellen dar:
- Figur 1: einen Querschnitt durch eine bevorzugte Ausführungsform einer erfindungsgemäßen Abziehvorrichtung mit einem Vorspannelement und mit einer Nadeleinheit am Injektionspen;
- Figur 2: einen Querschnitt der Ausführungsform aus Figur 1 in vorgespanntem Zustand,
- Figur 3: einen Querschnitt der Ausführungsform aus Figur 1 mit einer abgezogenen Nadeleinheit und
- Figur 4: ein System aus einem Behälter mit einer Nadelabziehvorrichtung und einem Container.

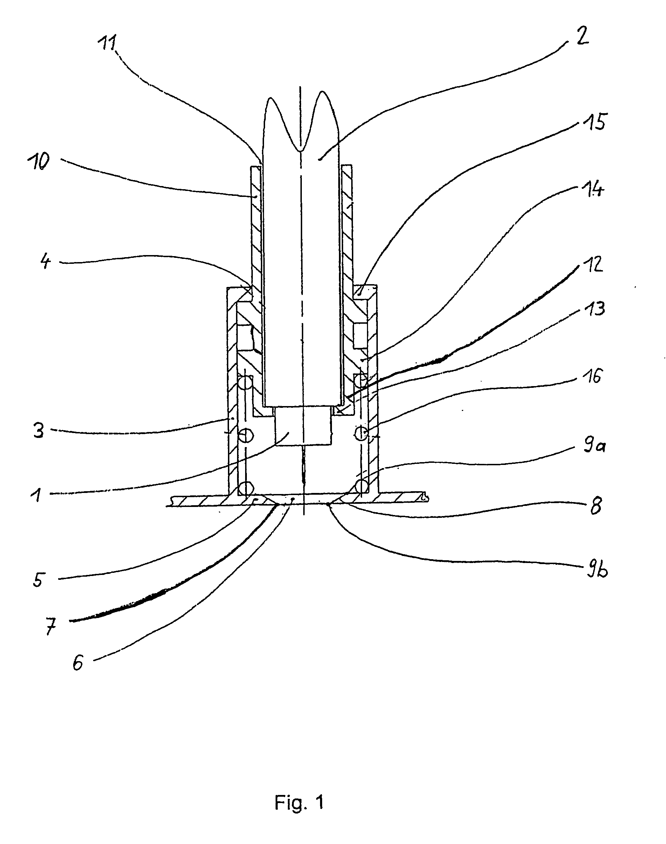

In Figur 1 ist eine Vorrichtung zum Abziehen einer Nadeleinheit 1 von einem Injektionspen 2 nach der vorliegenden Erfindung gezeigt, die ein zylinderförmiges Führungselement 3 aufweist. Das Führungselement 3 ist mit einer oberen Einführöffnung 4 zum Einführen des Injektionspens 2 und einem unteren Bereich 5 versehen, in dem eine Öffnung 6 eingebracht ist. In der Öffnung 6 ist erfindungsgemäß in einem Randbereich eine Sperreinrichtung 7 angeordnet. Die Sperreinrichtung 7 erstreckt sich über den gesamten Umfang des Randes 8 der Öffnung 6.

Die Sperreinrichtung 7 ist in diesem Ausführungsbeispiel durch einen sich konisch nach außen verengenden Rand 8 der Öffnung 6 gegeben, d. h. der Rand 8 der Öffnung 6 im Bereich 5 des Führungselements 3 weist an der innen liegenden Kante 9a einen größeren Umfang auf, als an der außen liegenden Kante 9b.

In dem Führungselement 3 ist ein Aufnahmerohr 10 für den Injektionspen 2 angeordnet, das innerhalb des Führungselements 3 verschiebbar ist. Ein oberes Ende 11 des Aufnahmerohrs 10 ragt durch die obere Öffnung 4 des Führungselements 3 aus dem Führungselement 3 heraus. Ein unteres Ende 12 liegt innerhalb des Führungselements 3 und weist bevorzugt nach innen gerichtete Vorsprünge 13 auf, an welchen der Injektionspen 2 beim Einsetzen in das Aufnahmerohr 10 aufsitzt. Die Nadeleinheit 1 des eingeführten Injektionspens 2 ragt an den Vorsprüngen 13 vorbei aus dem Aufnahmerohr 10 in Richtung auf die Sperreinrichtung 7 heraus.

Vorzugsweise sind seitlich am Außenumfang des Aufnahmerohres 10 Vorsprünge14 angeordnet, die an der Innenfläche des Führungselementes 3 anliegen. Die äußeren Vorsprünge 14 dienen zum eine der Führung des Aufnahmerohrs 10 in dem Führungselement 3. Zum anderen wirken sie als Widerlager einer Spiralfeder 16, die als Vorspannelement für das Aufnahmerohr 10 zwischen das Aufnahmerohr 10 und das Führungselement 3 eingesetzt ist. Das gegenüberliegende Widerlager für die Spiralfeder 16 wird von dem unteren Bereich 5 des Führungselements 3 gebildet. Die Windungen der Feder verlaufen an der Innenfläche des Führungselements 3.

In Figur 1 ist der Injektionspen 2 vollständig in das Aufnahmerohr 10 eingeführt, so dass er an den inneren Vorsprüngen 13 anstößt und die Nadeleinheit 1 in Richtung auf die Sperreinrichtung 7 positioniert. Die Feder 16 ist in einem entspannten Zustand und die äußeren Vorsprünge 14 stoßen an den nach innen ragenden Kanten 15 des Führungselements 3 an.

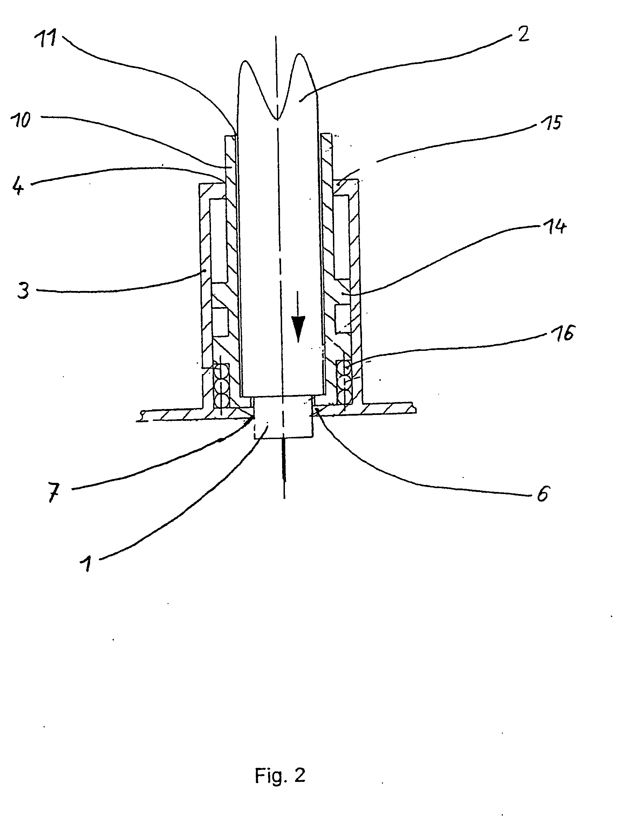

In Figur 2 ist die Abziehvorrichtung aus Figur 1 gezeigt, bei der nun die Nadeleinheit 1 durch die Öffnung 6 in die Sperreinrichtung 7 eingreift. Hierfür wurde das Aufnahmerohr 10 durch manuelle Kraft entgegen der Federkraft der Spiralfeder 16 innerhalb des Führungselements 3 in Richtung des gezeigten Pfeils verschoben bis das untere Ende 12 des Aufnahmerohrs 10 am unteren Bereich 5 des Führungselements 3 anstößt und die nach unten aus dem Aufnahmerohr 10 herausragende Nadeleinheit 1 durch die Öffnung 6 in die Sperreinrichtung 7 gepresst wird. Dadurch entsteht eine Sperrverbindung zwischen der Nadeleinheit 1 und der Sperreinrichtung 7. Die Spiralfeder 16 wird beim Eindrücken der Nadeleinheit 1 in die Sperreinrichtung 7 in einen vorgespannten Zustand versetzt.

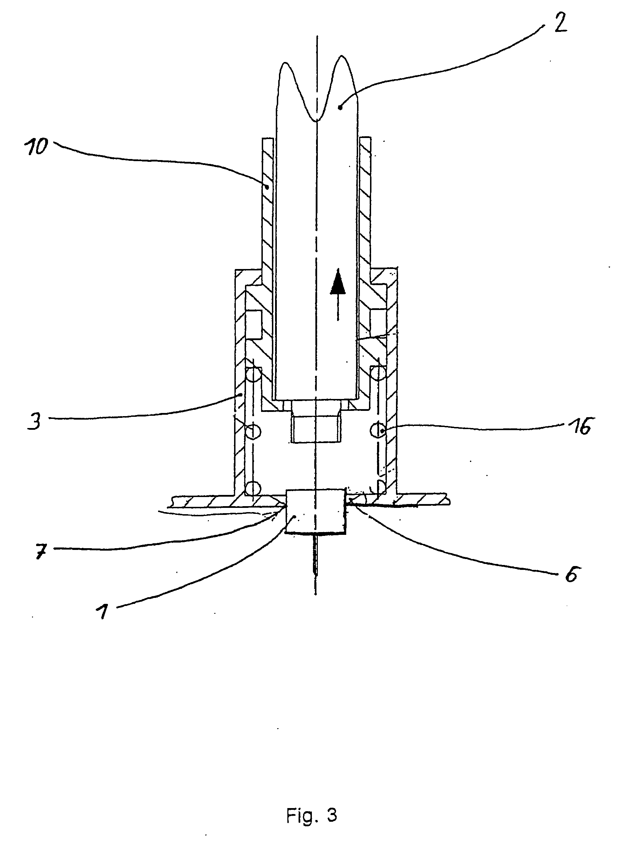

In Figur 3 ist die Abziehvorrichtung mit einer abgezogenen Nadeleinheit 1 dargestellt. Zum Abziehen der Nadeleinheit 1 von dem Injektionspen 2 ist es lediglich erforderlich, die manuell auf den Injektionspen 2 ausgeübte Kraft zu reduzieren, denn durch die Federkraft der Spiralfeder 16 wird das Aufnahmerohr 10 automatisch in Richtung des gezeigten Pfeils aus dem Führungselement 3 geschoben. Die Sperreinrichtung 7 wirkt wie Haltekrallen auf die Nadeleinheit 1, so dass diese bei der Rückbewegung des Injektionspens 2 von diesem abgezogen wird. Sie kann wie in Figur 3 dargestellt ist in der Sperreinrichtung 7 verbleiben und erst durch das Einführen eines weiteren Injektionspens durch dessen Nadeleinheit aus der Sperreinrichtung 7 gestoßen werden.

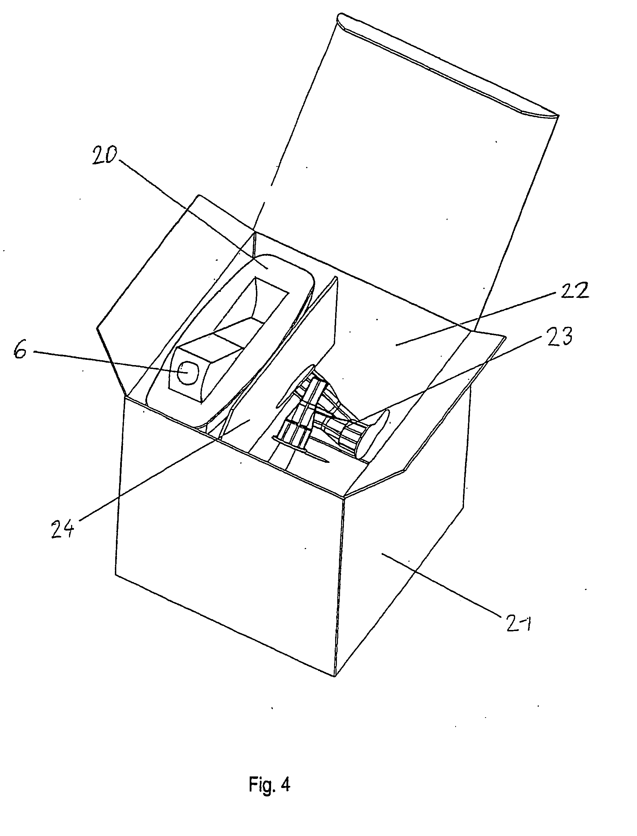

In Figur 4 ist ein System aus einem Behälter 20 und einem Container 21 gezeigt, der den Behälter 20 aufnimmt und einen Depotraum 22 für unbenutzte Nadeleinheiten 23 aufweist. Die Höhe des Containers 21 entspricht im wesentlichen der Höhe des Behälters 20. Eine Trennwand 24 sichert den Behälter 20 auf einer Seite des Containers 21. Der verbleibende Raum auf der anderen Seite des Containers 21 bildet den Depotraum 22. Der Behälter 20 weist in dem gezeigten Beispiel eine Klappevorrichtung auf, durch die eine Abziehvorrichtung mit einer Öffnung 6 und einem Sperrmittel gemäß der Erfindung herausgeklappt werden kann, so dass die Öffnung 6 von außen zugänglich wird. Der Behälter 20 muss deshalb zur Entsorgung einer gebrauchten Nadeleinheit nicht aus dem Container 21 herausgenommen werden. Wurde eine gebrauchte Nadeleinheit von einem Injektionspen mit Hilfe der Abziehvorrichtung des Behälters 20 in diesem entsorgt, kann die Klappvorrichtung wieder eingeklappt werden, so dass die Öffnung 6 der Abziehvorrichtung in den Behälter versenkt wird. Vorzugsweise ist der Behälter 20 fest verschlossen und lässt sich nicht öffnen, damit die gebrauchten Nadeleinheiten unzugänglich sind. In dem Depotraum 22 können so viele unbenutzte Nadeleinheiten 23 aufgenommen werden, wie nach deren Gebrauch in dem Behälter 20 entsorgt werden können.

Natürlich ist es auch möglich, das System aus Behälter und Container durch eine einheitliche Box zu bilden, bzw. einen Behälter, mit einem eigenen Depotraum zu versehen. Hierfür ist die Box bzw. der Behälter, durch eine Trennwand in zwei Bereiche unterteilt. Ein Bereich ist fest verschlossen und weist eine erfindungsgemäße Abziehvorrichtung, vorzugsweise als Teil einer Klappvorrichtung, für gebrauchte Nadeleinheiten auf. In dem anderen Bereich können die unbenutzten Nadeleinheiten untergebracht werden. Dieser Bereich ist mit Hilfe eines Deckels verschließbar, der sich jedoch öffnen lässt. Wiederum sind die Fassungsvolumina der beiden Bereiche derart aufeinander abgestimmt, dass die Anzahl unbenutzter Nadeleinheiten nach ihrem Gebrauch in dem verschlossenen Bereich mit der Abziehvorrichtung untergebracht werden können. Sind keine unbenutzten Nadeleinheiten mehr vorhanden, ist der Bereich für die benutzten Nadeleinheiten gefüllt und die Box, bzw. der Behälter, kann als ganzes entsorgt werden.

## Patentansprüche

1. Vorrichtung zum Abziehen einer Nadeleinheit (1) von einer Injektionsvorrichtung (2) mit einer Öffnung (6) zum Aufnehmen der Nadeleinheit (1) und mit einem Sperrmittel (7), wobei das Sperrmittel (7) in wenigstens einem Randbereich entlang des Umfangs der Öffnung (6) derart ausgebildet ist, dass die Nadeleinheit (1) nach dem Einführen in die Öffnung (6) in Einführrichtung beweglich ist und eine Bewegung der Nadeleinheit (1) entgegen der Einführrichtung gesperrt wird, wobei ein zylinderförmiges Führungselement (3, 10) mit einer Einführöffnung (4) für die Injektionsvorrichtung (2) über der Öffnung (6) angeordnet ist, **dadurch gekennzeichnet, dass** in dem Führungselement (3) ein Aufnahmerohr (10) für die Injektionsvorrichtung (2) verschiebbar angeordnet ist.

2. Vorrichtung nach Anspruch 1, **dadurch gekennzeichnet, dass** das Aufnahmerohr (10) aufweist:
- ein oberes Ende (11), das durch die Einführöffnung (4) aus dem Führungselement (3) herausragt und
- ein unteres Ende (12), das innerhalb des Führungselements (3) liegt und nach innen gerichtete Vorsprünge (13) aufweist, an welchen die Injektionsvorrichtung (2) aufsitzen kann, sodass die Nadeleinheit (1) an diesen Vorsprüngen (13) vorbei aus dem Aufnahmerohr (10) in Richtung der Sperreinrichtung (7) hinausragt.

3. Vorrichtung nach Anspruch 1 oder 2, **dadurch gekennzeichnet, dass** am Aussenumfang des Aufnahmerohrs (10) seitliche Vorsprünge (14) angeordnet sind, die an der innenfläche des Führungselementes (3) anliegen, sodass das Aufnahmerohr (10) von dem Führungselement (3) geführt ist.

4. Vorrichtung nach einem der vorhergehenden Ansprüche, **dadurch gekennzeichnet, dass** zwischen dem Aufnahmerohr (10) und dem Führungselement (3) ein Vorspannelement (16) angeordnet ist.

5. Vorrichtung nach dem Anspruch 4, **dadurch gekennzeichnet, dass** das Vorspannelement (16) durch eine Spiralfeder gegeben ist.

6. Vorrichtung nach einem der vorhergehenden Ansprüche, **dadurch gekennzeichnet, dass** in der Wand des Führungselements eine Hebelanordnung zur Betätigung des Aufnahmerohrs (10) vorgesehen ist.

7. Vorrichtung nach Anspruch 6, **dadurch gekennzeichnet, dass** durch die Wand des Führungselements (3) in einem unteren Bereich ein Hebel angeordnet ist, dessen innen liegendes Ende beim Eingreifen der Nadeleinheit (1) in die Sperreinrichtung (7) an dem Aufnahmerohr (10) angreift und dessen aussen liegendes Ende derart von aussen betätigbar ist, dass das Aufnahmerohr (10) aus dem Führungselement (3) schiebbar ist.

8. System mit einer Vorrichtung nach einem der vorhergehenden Ansprüche und einer Injektionsvorrichtung (2) mit einer Nadeleinheit (1)

9. System nach Anspruch 8, **dadurch gekennzeichnet, dass** das Rastmittel durch mehrere in der Randfläche der Öffnung (6) der Vorrichtung angeordnete Rillen und das komplementäre Rastmittel durch mehrere in Umfangsrichtung auf der Aussenfläche der Nadeleinheit (1) angeordnete Vorsprünge gegeben ist.

10. System nach Anspruch 9, **dadurch gekennzeichnet, dass** im Rand (8) der Öffnung (6) der Vorrichtung wenigstens eine zumindest teilweise schräg verlaufende Führungsrille mit einem Sperrbereich für die Nadeleinheit (1) vorgesehen ist, die an einer in Einführrichtung ersten Kante (9a) der Randfläche der Öffnung (6) beginnt und in eine zweite Kante (9b) der Öffnung (6) mündet, wobei an der Nadeleinheit (1) wenigstens ein Stift angeordnet ist, der in die wenigsten eine Führungsrille eingreift.

11. System nach einem der Ansprüche 8 bis 10, **dadurch gekennzeichnet, dass** sich nach dem Herausziehen der Injektionsvorrichtung (2) aus der Vorrichtung zum Abziehen der Nadeleinheit der Sperrverbindung der Nadeleinheit (1) mit der Sperreinrichtung (7) löst und die Nadeleinheit (1) freigegeben wird.

12. Behälter zur Aufbewahrung wenigstens einer gebrauchten Nadeleinheit (1), **dadurch gekennzeichnet, dass** er mit einer Vorrichtung zum Abziehen der Nadeleinheit (1) von einer Injektionsvorrichtung (2) nach einem der Ansprüche 1 bis 7 ausgebildet ist.

13. Behälter nach Anspruch 12, **dadurch gekennzeichnet, dass** er einen Depotraum für unbenutzte Nadeleinheiten (1) umfasst, der von einem Raum mit der Abziehvorrichtung für gebrauchte Nadeleinheiten (1) getrennt ist.

## Claims

1. A device for removing a needle unit (1) from an injection apparatus (2) comprising an opening (6) for receiving the needle unit (1) and a locking means (7), wherein the locking means (7) is provided in at least one edge region along the periphery of the opening (6) in such a way that after insertion into the opening (6) the needle unit (1) is movable in the insertion direction and a movement of the needle unit (1) in opposite relationship to the insertion direction is locked, wherein a cylindrical guide element (3, 10) with an insertion opening (4) for the injection apparatus (2) is arranged over the opening (6), **characterised in that** a receiving tube (10) for the injection apparatus (2) is arranged displaceably in the guide element (3).

2. A device according to claim 1 **characterised in that** the receiving tube (10) has:
- an upper end (11) which projects out of the guide element (3) through the insertion opening (4), and
- a lower end (12) which is arranged within the guide element (3) and has inwardly directed projections (13) on which the injection apparatus (2) can sit so that the needle unit (1) projects past those projections (13) out of the receiving tube (10) in the direction of the locking means (7).

3. A device according to claim 1 or claim 2 **characterised in that** arranged on the outer periphery of the receiving tube (10) are lateral projections (14) which bear against the inside surface of the guide element (3) so that the receiving tube (10) is guided by the guide element (3).

4. A device according to one of the preceding claims **characterised in that** a biasing element (16) is arranged between the receiving tube (10) and the guide element (3).

5. A device according to claim 4 **characterised in that** the biasing element (16) is provided by a coil spring.

6. A device according to one of the preceding claims **characterised in that** provided in the wall of the guide element is a lever arrangement for actuation of the receiving tube (10).

7. A device according to claim 6 **characterised in that** a lever is arranged through the wall of the guide element (3) in a lower region, the inwardly disposed end of which lever engages the receiving tube (10) when the needle unit (1) engages into the locking means (7) and the outwardly disposed end of the lever being actuable from the exterior in such a way that the receiving tube (10) can be pushed out of the guide element (3).

8. A system comprising a device according to one of the preceding claims and an injection apparatus (2) having a needle unit (1).

9. A system according to claim 8 **characterised in that** the latching means is provided by a plurality of grooves arranged in the edge surface of the opening (6) of the device and the complementary latching means is provided by a plurality of projections arranged in the peripheral direction on the outside surface of the needle unit (1).

10. A system according to claim 9 **characterised in that** provided in the edge (8) of the opening (6) of the device is at least one at least partially inclinedly extending guide groove with a locking region for the needle unit (1), which begins at an edge (9a) which is the first edge in the insertion direction of the edge surface of the opening (6) and which passes into a second edge (9b) of the opening (6), wherein arranged on the needle unit (1) is at least one pin which engages into the at least one guide groove.

11. A system according to one of claims 8 to 10 **characterised in that** after the injection apparatus (2) has been pulled out of the device for removal of the needle unit the locking connection of the needle unit (1) to the locking means (7) is released and the needle unit (1) is freed.

12. A container for storing at least one used needle unit (1) **characterised in that** it is provided with a device for removing the needle unit (1) from an injection apparatus (2) according to one of claims 1 to 7.

13. A container according to claim 12 **characterised in that** it includes a repository space for unused needle units (1), which is separated from a space with the removal device for used needle units (1).

## Revendications

1. Dispositif de retrait d'une d'aiguille (1) d'un dispositif d'injection (2) doté d'une ouverture (6) pour recevoir l'aiguillé (1) et d'un moyen de blocage (7), le moyen de blocage (7) étant agencé dans au moins une zone périphérique le long du pourtour de l'ouverture (6) de telle sorte que l'aiguille (1) soit mobile après l'introduction dans l'ouverture (6) dans le sens d'introduction et qu'un mouvement de l'aiguille (1) à l'opposé du sens d'introduction soit bloqué, un élément de guidage cylindrique (3, 10) avec une ouverture d'introduction (4) pour le dispositif d'injection (2) étant disposé au dessus de l'ouverture (6), **caractérisé en ce qu'**un tube de réception (10) pour le dispositif d'injection (2) est disposé dans l'élément de guidage (3), de façon à pouvoir coulisser.

2. Dispositif selon la revendication 1, **caractérisé en ce que** le tube de réception (10) présente :
une extrémité supérieure (11) qui dépasse de l'élément de guidage (3) par l'ouverture d'introduction (4) et
une extrémité inférieure (12) qui se situe à l'intérieur de l'élément de guidage (3) et présente des proéminences (13) dirigées vers l'intérieur sur lesquelles le dispositif d'injection (2) peut reposer de sorte que l'aiguille (1) dépasse au niveau de ces proéminences (13) hors du tube de réception (10) dans la direction du dispositif de blocage (7).

3. Dispositif selon la revendication 1 ou 2, **caractérisé en ce que** des proéminences latérales (14) sont disposées sur le pourtour externe du tube de réception (10), lesquelles reposent sur la surface interne de l'élément de guidage (3) de sorte que le tube de réception (10) soit guidé par l'élément de guidage (3).

4. Dispositif selon l'une quelconque des revendications précédentes, **caractérisé en ce qu'**est disposé un élément de précontrainte (16) entre le tube de réception (10) et l'élément de guidage (3)

5. Dispositif selon la revendication 4, **caractérisé en ce que** l'élément de précontrainte (16) est réalisé par un ressort à spirales.

6. Dispositif selon l'une quelconque des revendications précédentes, **caractérisé en ce qu'**un dispositif de levier pour actionner le tube de réception (10) est prévu dans la paroi de l'élément de guidage.

7. Dispositif selon la revendication 6, **caractérisé en ce que**, au travers de la paroi de l'élément de guidage (3) est disposé dans une zone inférieure, un levier dont l'extrémité reposant à l'intérieur agit lors de la mise en prise de l'aiguille (1) dans le dispositif de blocage (7) sur le tube de réception (10) et dont l'extrémité située à l'extérieur peut être actionnée de telle sorte que le tube de réception (10) puisse coulisser hors de l'élément de guidage (3).

8. Système doté d'un dispositif selon l'une quelconque des revendications précédentes et d'un dispositif d'injection (2) avec une aiguille (1).

9. Système selon la revendication 8, **caractérisé en ce que** le moyen d'arrêt est doté de plusieurs sillons disposés dans la surface périphérique de l'ouverture (6) du dispositif et le moyen d'arrêt complémentaire est doté de plusieurs proéminences disposées suivant une direction périphérique sur la surface extérieure de l'aiguille (1).

10. Système selon la revendication 9, **caractérisé en ce que**, dans la périphérie (8) de l'ouverture (6) du dispositif est prévu au moins un sillon de guidage évoluant en partie obliquement avec une zone de blocage pour l'aiguille (1) qui commence à un premier bord (9a) de la surface périphérique de l'ouverture (6) dans le sens d'introduction et débouche dans un deuxième bord (9b) de l'ouverture (6), au moins une pointe étant aménagée au niveau de l'aiguille (1) qui est en engagement dans au moins un sillon de guidage.

11. Système selon l'une quelconque des revendications 8 à 10, **caractérisé en ce que**, après le retrait du dispositif d'injection (2) du dispositif pour le retrait de l'aiguille, le blocage de l'aiguille (1) avec le dispositif de blocage (7) est débloqué et l'aiguille (1) est libérée.

12. Conteneur pour conserver au moins une aiguille usagée (1), **caractérisé en ce qu'**il est réalisé avec un dispositif de retrait de l'aiguille (1) d'un dispositif d'injection (2) selon l'une des revendications 1 à 7.

13. Conteneur selon la revendication 12, **caractérisé en ce qu'**il comprend un espace de dépôt d'aiguilles non utilisées (1) qui est séparé d'un espace présentant un dispositif de retrait pour les aiguilles usagées.
